# EUROPEAN PATENT SPECIFICATION

(11) **EP 4 554 926 B1**
(45) Date of publication and mention of the grant of the patent: **09.09.2026**
(21) Application number: 23741720.9
(22) Date of filing: 14.07.2023
(51) Int. Cl.: C07C 67/297, C07C 69/76

(54) **PROCESS FOR PREPARING BENZOIC ACID ESTERS AND INTERMEDIATES THEREOF**
VERFAHREN ZUR HERSTELLUNG VON BENZOESÄUREESTERN UND DEREN ZWISCHENPRODUKTEN
PROCÉDÉ DE PRÉPARATION D'ESTERS D'ACIDE BENZOÏQUE ET INTERMÉDIAIRES CORRESPONDANTS

(30) Priority: 15.07.2022 EP 22382681
(43) Date of publication of application: 21.05.2025
(73) Proprietor: Roka Furadada, SL, 08030 Barcelona (ES)
(72) Inventor: ATCHER UBIERGO, Joan, 08030 BARCELONA (ES); CAMARGO SANROMÀ, Judit, 08030 BARCELONA (ES); TORTOSA I PERPINYÀ, Arturo, 08030 BARCELONA (ES); VALDIVIELSO PABLO, Ángel Manuel, 08030 BARCELONA (ES)
(74) Representative: ZBM Patents - Zea, Barlocci & Markvardsen
(86) International application number: PCT/EP2023/069683
(87) International publication number: WO 2024/013382

(56) References cited:
- EP-A1- 1 707 558
- QI XINXIN ET AL: "Palladium/aluminium-cocatalyzed carbonylative synthesis of 2-chloroethyl benzoates from epoxides and aryl iodides", JOURNAL OF ORGANOMETALLIC CHEMISTRY, ELSEVIER, AMSTERDAM, NL, vol. 910, 16 January 2020 (2020-01-16), XP086041059, ISSN: 0022-328X, [retrieved on 20200116], DOI: 10.1016/J.JORGANCHEM.2020.121114
- OKAMOTO NORIKO ET AL: "Regio- and Stereoselective Multisubstituted Enol Ester Synthesis", THE JOURNAL OF ORGANIC CHEMISTRY, vol. 76, no. 21, 6 October 2011 (2011-10-06), pages 9133 - 9138, XP093011169, ISSN: 0022-3263, DOI: 10.1021/jo201609r

## Description

This application claims the benefit of European Patent Application EP22382681 filed 15 July 2022.

### Field of the invention

The present invention relates to a process for the preparation of benzoic acid ester compounds, as well as to some new intermediates useful in such preparation process.

### Background Art

Several benzoic acid esters have been described as photochemical precursors of ultraviolet absorbers. Their photoprotective activity is due to the fact that are susceptible to be photochemically converted *in situ* to a sunscreen compound with enhanced UV protection ability.

Compounds of formula (I) can be obtained by a great variety of methods disclosed in the art. Thus, WO2006/100225A1 discloses benzoic acid ester compounds and processes for their preparation based on the reaction of an acyl halide with an intermediate silyl enol ether according to the following scheme, in which R1-R21 and R' have defined values.

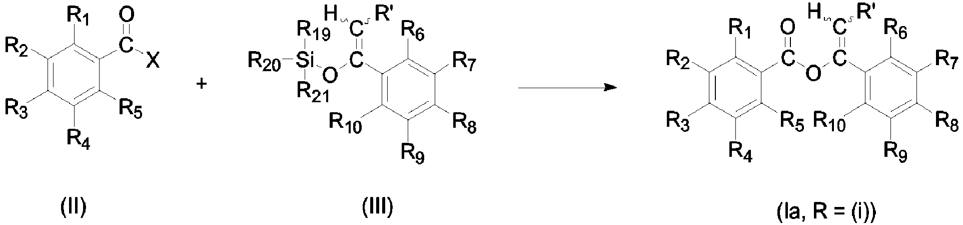

Another process for the preparation of a specific benzoic acid ester compound, 1-(4-methoxyphenyl) vinyl 4-(tert-butyl) benzoate, is disclosed in *"*Dose-dependent progressive sunscreens. A new strategy for photoprotection?', Photochemical & Photobiological Sciences (2010), 9(4), 530-534 **(D2),** the following scheme illustrates the process:

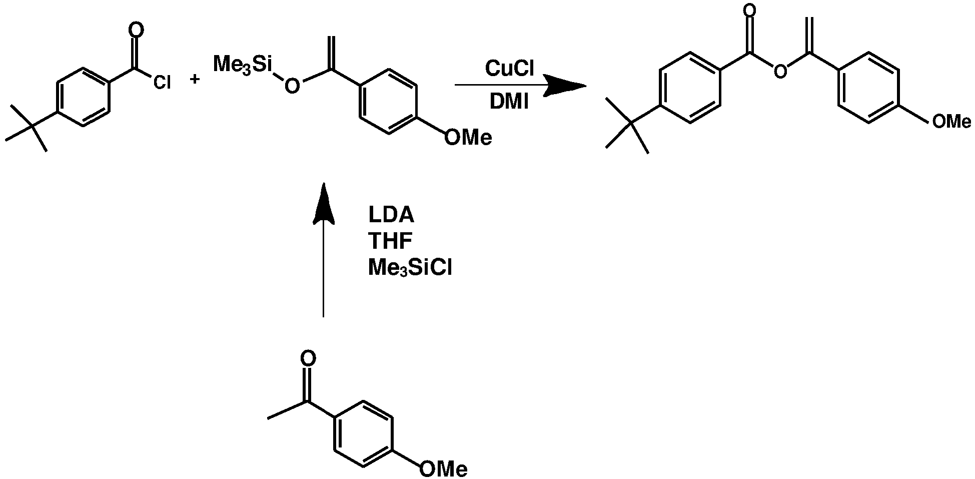

Another process for preparing some benzoic acid ester compounds is disclosed by Noriko et al., and relates to a multisubstituted enol ester synthesis based on reaction of alkynes having aromatic substituents with NXS being X selected from Cl, Br, and I, and AcOH, followed by a coupling reaction (cf. Noriko Okamoto et al, " Regio- and Stereoselective Multisubstituted Enol Ester Synthesis" JOC, 2011, vol. 76, pp. 9133-9138).

A preparation process of 2-chloroethyl benzoates based on palladium/aluminium-cocatalyzed carbonylative synthesis of 2-clhoroetyhl benzoates from epoxides and aryl iodides have been disclosed in the art (cf. Qi Xinxin et al.; "Palladium/aluminium-cocatalyzed carbonlylative synthesis of 2-chloroethyl benzoates from epoxides and aryl iodides", Journal of Organometallic Chemistry, 2020, vol. 910, 121114)

However, the research of new preparation processes of these benzoic acid esters with good yields and purity and that can be carried out at industrial scale is still an active field, Thus, the provision of new preparation processes of such compounds is highly desirable.

### Summary of Invention

Inventors have found a new process for the preparation of benzoic acid esters which proceeds with good yields, good purity, and can be conducted at industrial scale Accordingly, a first aspect of the present invention relates to a process for preparing a compound of formula (I), a pharmaceutically or a cosmetically acceptable salt thereof, or a stereoisomer of any of them or mixtures thereof; 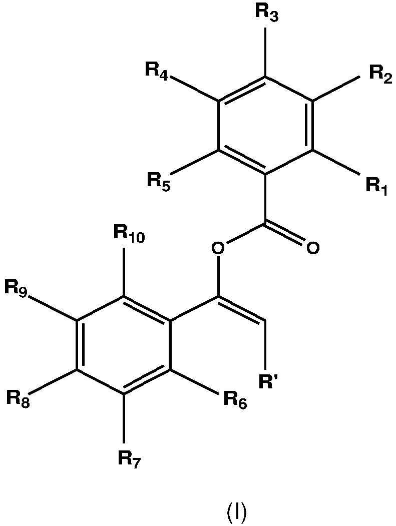 wherein: R' is selected from the group consisting of **H,** (C₁-C₆)-alkyl, and (C₃-C₆)-cycloalkyl; R₁, R₂, R₄, R₅, R₆, R₇, R₉, and R₁₀ are a radical independently selected from the group consisting of **H,** hydroxy, amino, (C₁-C₆)-alkyl, (C₁-C₆)-alkoxy, (C₁-C₆)-alkylamino, and (C₁-C₆)-dialkylamino;; and R₃,and R₈ are independently selected from the group consisting of (C₁-C₆)-alkyl, (C₁-C₆)-alkoxy, hydroxy, amino, (C₁-C₆)-alkylamino, and (C₁-C₆)-dialkylamino; the process comprising a step of reacting a compound of formula (II) or an appropriate salt thereof, wherein: R', R_{1 -} R₁₀, are as defined in compound of formula (I) and X is a halogen, with a non-nucleophilic strong base, in the presence of an appropriate solvent,

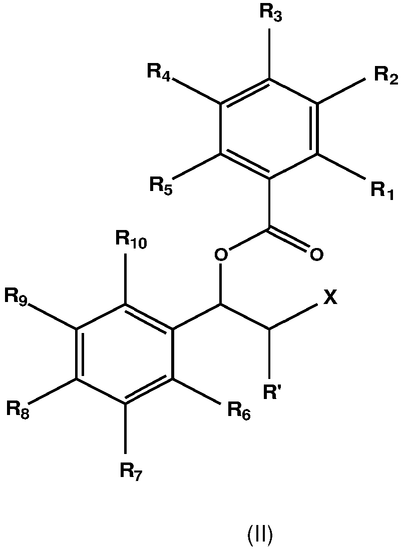

Another aspect of the present invention relates to a compound of formula (II) above or a salt thereof, wherein: R' is H; R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, (C₁-C₆)-alkylamino, and (C₁-C₆)-dialkylamino; R₃ and R₈ are independently selected form the group consisting of (C₁-C₆)-alkyl, (C₁-C₆)-alkoxy, hydroxy, amino, (C₁-C₆)-alkylamino, and (C₁-C₆)-dialkylamino; and X is a halogen selected from the group consisting of Cl, Br, and I.

### Detailed description of the invention

For the purposes of the invention, any ranges given include both the lower and the upper endpoints of the range. Ranges given, such as temperatures, times, sizes, and the like, should be considered approximate, unless specifically stated.

The term "room temperature" as disclosed herein refers to a temperature of the environment, without heating or cooling, and is generally comprised of from 20 to 25 ºC.

Throughout the description and claims, the terms (C₁-C₆)-alkyl, (C₁-C₆)-alkoxy, (C₁-C₆)-alkylamino, and (C₁-C₆)-dialkylamino shall be construed as straight or branched.

As mentioned above, it is part of the invention a process for preparing a compound of formula (I), a pharmaceutically or a cosmetically acceptable salt thereof, or a stereoisomer of any of them or mixtures thereof, wherein: R' is is selected from the group consisting of H, (C₁-C₆)-alkyl, and (C₃-C₆)-cycloalkyl; R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, C₁-C₆)-alkylamino, and (C₁-C₆)-dialkylamino; and R₃ and R₈, are independently selected form the group consisting of (C₁-C₆)-alkyl, (C₁-C₆)-alkoxy, hydroxy, amino, (C₁-C₆)-alkylamino, and (C₁-C₆)-dialkylamino; the process comprising a step of reacting a compound of formula (II) wherein: R', R₁-R₁₀, are as defined in compound of formula (I) and X is a halogen as defined above, with a non-nucleophilic strong base, in the presence of an appropriate solvent.

Compounds of formula (I) when R' is different to H exhibit cis-trans isomerism. The process according to the invention for preparing any of the isomers of the compound of formula (I) or mixtures thereof as defined above forms part of the invention. Generally, a mixture of isomers is obtained. When desired, the isomers can be separated by conventional means of purification.

In a particular embodiment, the process is that in which in compounds of formula (I), R' is H.

In another particular embodiment, the process is that where in compound of formula (I) and in compound of formula (II) R₁, R₂, R₄, R₅, R₆, R₇, R₉, and R₁₀ are a radical independently selected from the group consisting of H, hydroxy, amino, and methyl.

In another particular embodiment, the process is that where in compound of formula (I) and in compound of (II), R₂, R₄, R₇, and R₉ are H.

In another particular embodiment, the process is that where in compound of formula (II), X is bromine. In another particular embodiment, the process is that where in compound of formula (I) and in compound of formula (II) R', R₁, R₂, R₄, R₅, R₆, R₇, R₉, and R₁₀ are H; R₃ is tert-butyl and R₈ is methoxyl. The preferred compounds produced by the process of the present invention are the following: 1-phenylvinyl 4-methoxybenzoate; 1-(4-methoxyphenyl)-vinyl 4-tert-butylbenzoate; 1-(4-tert-butylphenyl) vinyl 4-methoxybenzoate; and 1-phenylvinyl 4-tert-butylbenzoate.

A non-nucleophilic strong base as used herein refers to a sterically hindered organic base that is a poor nucleophile. Typical non-nucleophilic bases are bulky, such that protons can attach to the basic center but alkylation and complexation is inhibited. Examples of non-nucleophilic strong bases are, 1,8-diazabicycloundec-7-ene (DBU), 1,1,3,3-Tetramethylguanidine (TMG), 1,5-diazabicyclo(4.3.0)non-5-ene (DBN), lithium diisopropylamide; silicon-based amides, such as sodium and potassium bis(trimethylsilyl)amide (NaHMDS and KHMDS, respectively), lithium tetramethylpiperidide (LiTMP). Other non-nucleophile strong bases may be inorganic bases such as sodium hydride, and potassium hydride.

In a particular embodiment, the non-nucleophilic strong base is selected from 1,8-diazabicyclo[5.4.0]undec-7-ene (DBU, Illa) and 1,1,3,3-tetramethylguanidine (TMG, Illb).

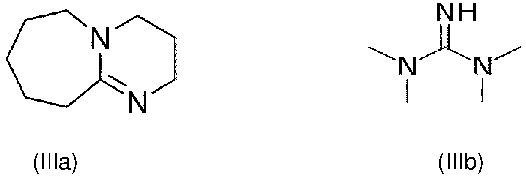

Generally, the amount of non-nucleophilic strong base is comprised in the range of 1 to 3 equivalents per equivalent of compound (II).

Appropriate solvents for the reaction are for instance (C₆-C₈)-aromatic hydrocarbons such as toluene or xylene, or (C₁-C₃)-chlorine containing solvents such as dichloromethane or dichloroethane.

Generally, the reaction is carried out at a temperature comprised in a range from 20 to 90 ºC. In a particular embodiment, the reaction is carried out at a temperature comprised in the range of 60-80 ºC. In another particular embodiment, the reaction is carried out at a temperature comprised between 70-75 ºC.

The term "appropriate salt" refers to a non-toxic salt. As some of the compounds of formula (I) are basic compounds, salts may be prepared with pharmaceutically or cosmetically acceptable non-toxic acids

Compound of formula (II) may also be in the form of a non-toxic salt. In a particular embodiment, the salt is a pharmaceutically or cosmetically acceptable salt as in compound of formula (I).

The term "pharmaceutically or cosmetically acceptable salt" used herein for any of compounds (I) and (II) encompasses any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5- naphthalendisulfonic, oxalic, pivalic, propionic, p- toluenesulfonic, succinic, tartaric acids and the like.

The preparation of pharmaceutically or cosmetically acceptable salts of the compounds of formula (I) or of the compound of formula (II) can be carried out by methods known in the art. For instance, they can be prepared from the parent compound, which contains a basic moiety, by conventional chemical methods. Generally, such salts are, for example, prepared by reacting the free base form of these compounds with a stoichiometric amount of the appropriate pharmaceutically or cosmetically acceptable acid in water or in an organic solvent or in a mixture of them.

When a non-toxic salt of compound of formula (II) is used to prepare a compound of formula (I), if desired, it can previously be converted in the free base by reaction with an appropriate base Alternatively, it can be used in the reaction in the form of a salt.

The compounds of formula (I) may be in crystalline form either as free solvation compounds or as solvates (e.g., hydrates) and it is intended that both forms are within the scope of the present invention. Methods of solvation are generally known within the art.

The term "solvate" refers to a molecular complex comprising the compound of formula (I) or a salt thereof, and a stoichiometric or non-stoichiometric amount of one or more solvent molecules bound by non-covalent intermolecular forces. When the one or more solvent molecules forming part of the molecular complex is water, the solvate is a hydrate.

Compound of formula (I) or its salts can be further purified for instance by crystallization in an appropriate solvent. Examples of appropriate solvent are (C₂-C₆)-alcohol such as ethanol, or 2-propanol. Compound of formula (I) can be obtained with a purity equal to or higher than 99% by HPLC. In a particular embodiment, compound of formula (I) is obtained with a purity equal to or higher than 99.5%. Other appropriate solvent can for the purification step can be (C₆-C₈)-aromatic hydrocarbons such as toluene or xylene, (C₁-C₃)-chlorine containing solvents such as dichloromethane, dichloroethane, or mixtures of (C₆-C₈)-aromatic hydrocarbons and (C₂-C₆)-alcohols. In a particular embodiment, the solvent mixture is 2-propanol/toluene.

Compound (II) can be prepared by a process comprising reacting a compound of formula (IV) with either a compound of formula (V) below or a stereoisomer thereof or mixtures thereof, in the presence of an appropriate solvent, where R', R₁-R₁₀ are as defined in compound of formula (I) and X is a halogen selected from the group consisting of Cl, Br, and I.

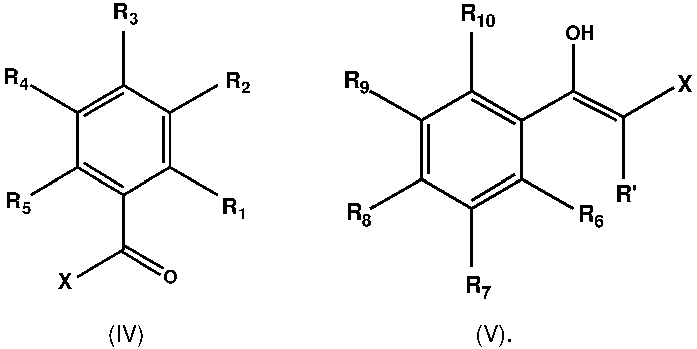

Thus, compounds of formula (V) when R' is different to H also exhibit cis-trans isomerism. The process according to the present invention for preparing compounds of formula (II) from any of the isomers of the compound of formula (V) or mixtures thereof as defined above, forms part of the invention. As for compound of formula (I), generally, a mixture of isomers is obtained. When desired, the isomers can be separated by conventional means of purification.

In a particular embodiment, the process for preparing compound of formula (II) is that which is carried out in the presence of a coupling agent capable of intervening in the conversion of an acyl halide to an ester. In a particular embodiment, the coupling agent is selected from the group consisting of 4-dimethylaminopyridine (DMAP), pyridine, 4-pyrrolidinopyridine (PPY), collidine isomers i.e., trimethyl derivatives of pyridine such as 3,4,5-collidine, 2,3,4-collidine, 2,3,5-collidine, 2,3,6 collidine. 2,4,5-collidine, 2,4,6-collidine, and 3,4,5-collidine, and N-methylimidazole. In another particular embodiment, the coupling agent is N,N-dimethylaminopyridine (DMAP).

Generally, an amount comprised in a range of 1 to 1.5 equivalents of coupling agent is used. In a particular embodiment, this step is carried out in the presence of an amount comprised in the range of 1.2-1.5 equivalents per equivalent of the compound of formula (IV). Optionally, a tertiary amine can also be present which may aid in regenerating the catalyst *in situ.* In a particular embodiment, an amount of DMAP of 1 equivalent is used and a tertiary amine is also present. An example of appropriate tertiary amine is triethylamine. When a tertiary amine is used, the DMAP can be used in substoichiometric amount such as 0.2 equivalents.

Appropriate solvents for the reaction are for instance (C₆-C₈)-aromatic hydrocarbons such as toluene or xylene, and (C₁-C₃)-chlorine containing solvents such as dichloromethane, or dichloroethane.

Generally, the reaction is carried out at a temperature comprised in a range from 0 ºC to 50 ºC. In a particular embodiment, the reaction is carried out at a temperature comprised in a range from 10-40 ºC. In another particular embodiment, the reaction is carried out at a temperature comprised in a range from 25-40 ºC.

Compound or formula (IV) can be obtained by a process comprising reacting a compound of formula (VI) below with a halogenating agent in the presence of an appropriate solvent, where R₁, R₂, R₃, R₄, and R₅, are as defined in compound of compound of formula (I).

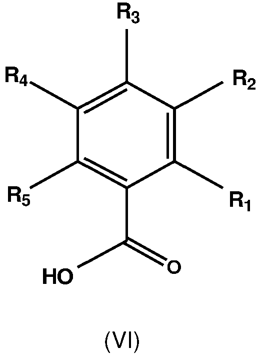

The halogenating agent can be selected from the group consisting of SO₂Cl₂, SOCl₂, PBr₃, and PBr₅ yielding to a compound of formula (IV) where X is Cl or Br. In a particular embodiment, the halogenating agent is SOCl₂.

The acyl chlorides can react with HF to give acyl fluorides. They can also be prepared directly from carboxylic acids, using PPh₃, NBS and Et₃N-3HF. Acyl iodides can be obtained for instance by reaction of acetyl iodide with monocarboxylic acids.

Appropriate solvents are, for instance, (C₆-C₈)-aromatic hydrocarbons such as toluene or xylene, or (C₁-C₃)-chlorine containing solvents such as dichloromethane or dichloroethane.

The reaction of the compound of formula (VI) with a halogenating agent is generally carried out at a temperature comprised in a range of from 50 ºC to 120 ºC. In a particular embodiment, the reaction is conducted at a temperature comprised in a range from 60 to 90 ºC. In another particular embodiment, the reaction is conducted at a temperature comprised in a range from 70 to 90 ºC.

Once the reaction is stopped, the excess of halogenating agent can be separated for instance by successive cycles of toluene addition followed by its distillation. The product can also be purified, for instance, by distillation.

Compound of formula (V) in any of their isomeric forms or mixtures thereof can be prepared by a process comprising reacting a compound of formula (VII) with a reducing agent in the presence of a solvent to yield the compound of formula (V), where: R', R₆, R₇, R₈, R₉, and R₁₀ are as defined in any of the embodiments of compound of formula (I) and X is a halogen.

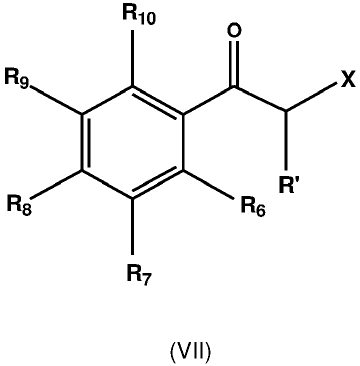

Appropriate solvents are, for instance protic solvents, such as (C₁-C₆)-alcohols. In a particular embodiment, the solvent is methanol. In another particular embodiment, the reducing agent used in the process for preparing compound (V) is sodium borohydride or lithium aluminum hydride.

The reaction of the compound of formula (VII) with a reducing agent is generally carried out at a temperature comprised in a range of from -10 ºC to 15 ºC. In a particular embodiment, the reaction is conducted at a temperature comprised between -10 ºC to 0 ºC. In a particular embodiment, the reaction is conducted at a temperature comprised between -10-(-5) ºC.

The product can be isolated carrying out extractions with a solvent such as toluene, followed by evaporation to dryness. Alternatively, the organic solution can be used directly in the next step, preferably, the water that can be present in the solution is removed, for instance, by evaporation.

The process may comprise carrying out the preparation step for preparing compound (VII) and a further step for its transformation in compound (II) without isolating the compound of formula (VII).

Some intermediates (IV) to (VII) to prepare the compound of formula (I) apart from compound of formula (II), may also be in the form of a non-toxic salt. The salts may be as those disclosed above. In a particular embodiment, the intermediates (IV) to (VII) are used in the form of a free base.

The preparation of salts of these intermediates that intervene in the process of the invention can be carried out by methods known in the art. For instance, they can be prepared from the parent compound, which contains a basic moiety, by conventional chemical methods. Generally, such salts are, for example, prepared by reacting the free base forms of these compounds with a stoichiometric amount of the appropriate non-toxic acid in an appropriate solvent.

With regard to the specific conditions for carrying out the steps of the process of the invention, the skilled person would know how to adjust the parameters of each of the steps indicated above in the light of the description and examples of the present invention.

It is part of the present invention the provision of single reaction step for preparing compound (I) from compound (II) of the global process of the present invention, as well as the combination of two or more sequential steps of the global process which include the step of transforming compound (II) into compound (I).

Scheme I illustrate the global process for preparing a specific compound of formula (I) wherein R', R₁, R₂, R₄, R₅, R₆ R₇, R₉, and R₁₀ are H; R₃ is tert-butyl, and R₈ is methoxyl.

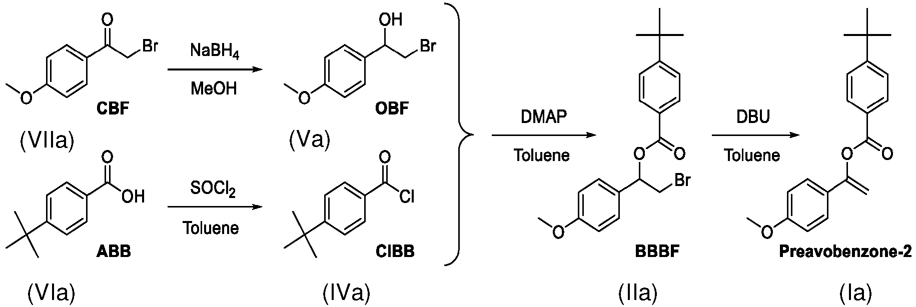

The process of the present invention can be carried out in sequential steps isolating the intermediates obtained, or alternatively, some of the steps of the present invention may be carried out in one pot.

Intermediate compounds of formula (II) or their salts mentioned above are new. Thus, the provision of the new compounds of formula (II) or their salts, which are useful intermediates for the preparation of benzoic acid esters according to the invention also forms part of the present invention.

It is considered part of the invention, the use of the compound of formula (II) as an intermediate to prepare compounds of formula (I) or a salt thereof. The intermediate compound of formula (II) as product per se is also part of the invention. In formula (II): R' is selected from the group consisting of H, (C₁-C₆)-alkyl, and (C₃-C₆)-cycloalkyl; R₁, R₂, R₄, R₅, R₆, R₇, R₉, and R₁₀, are a radical independently selected from the group consisting of: H, hydroxy, amino, (C₁-C₆)-alkyl, (C₁-C₆)-alkoxy, (C₁-C₆)-alkylamino, and (C₁-C₆)-dialkylamino; R₃ and R₈ are independently selected from the group consisting of (C₁-C₆)-alkyl, (C₁-C₆)-alkoxy, hydroxy, amino, (C₁-C₆)-alkylamino, and (C₁-C₆)-dialkylamino; and X is a halogen selected from the group consisting of: Cl, Br, and I.

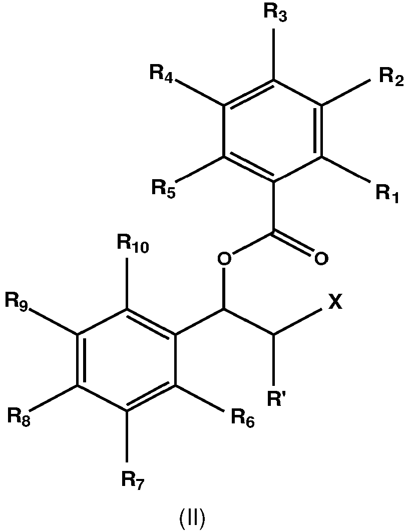

The term halogen means any of Cl, Br, and I. In a particular embodiment, compound of formula (II) is that, wherein X is bromine. In another particular embodiment, the compound of formula (II) is that where R₃ is tert-butyl. In another particular embodiment, the compound of formula (II) is that wherein R₈ is methoxyl. In another particular embodiment, the compound of formula (II) is that wherein R' is H. In another particular embodiment, the process is that where in compound of formula (I) and in compound of formula (II) R₁, R₂, R₄, R₅, R₆, R₇, R₉, and R₁₀ are a radical independently selected from the group consisting of H, hydroxy, amino, and methyl. In another particular embodiment, the process is that where in compound of formula (I) and compound of (II), R₂, R₄, R₇, and R₉ are H. In another particular embodiment, the compound of formula (II) is that wherein R₁, R₅, R₆, and R₁₀ are H.

The combination of particular embodiments of the product between them are also embodiments for the products of compound (II) according to the present invention. In a particular embodiment, the compound of formula (II) is that wherein R₃ is tert-butyl and R₈ is methoxyl. In another particular embodiment, the compound of formula (II) is that wherein R', R₁, R₂, R₄, R₅, R₆, R₇, R₉, and R₁₀ are H.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1: Preparation of: 2-bromo-1-(4-methoxyphenyl)ethan-1-ol (OBF)

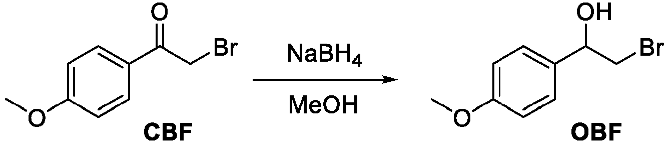

CBF (100 g, 0.437 mol) was suspended in MeOH (370 mL) in a 2 L reactor equipped with mechanical stirring and a dropping funnel with pressure compensation, under inert atmosphere of N2. The suspension was cooled down to -5 °C and stirred for 15 min.

A solution of NaBH₄ (16.55 g, 0.437 mol) and NaOH (80 mg) in MeOH (160 mL) at (-10)-(-3) °C was added during 1.5-2.0 h into the reactor, keeping the temperature of the resulting mixture at (-10)-(-3) °C. Once the addition was finished, the reaction mixture was further stirred at (-10)-(-3) °C for 5-10 min, after which complete conversion of the starting material was observed by TLC. DCM (500 mL) was added into the reactor during 30 min, keeping the temperature at (-10)-(-2) °C. The resulting mixture was stirred at (-10)-(-2) °C for 5 min. H2O (400 mL) was added into the reactor during 45 min, keeping the temperature at (-5)-0 °C. The resulting mixture was stirred at (-5)-0 ºC for 10 min. The mixture was acidified from pH 11-12 to pH 1-2 by adding 85 mL of 6 M aqueous HCl into the reactor during 30 min, keeping the temperature at (-5)-(-3) °C. The resulting mixture was stirred at (-5)-(-3) °C for 10 min. The stirring was stopped, and the mixture was let to decant for 15 min. The organic phase was washed with 3 x 250 mL of water, keeping the temperature at 10-15 ºC. The organic phase was dried over 20 g of MgSO₄, stirring the suspension at room temperature for 1 h. The solution was filtered and concentrated under reduced pressure at 20-25 ºC for approximately 6 h, until constant weight, obtaining 91.23 g (90% yield) of OBF as a brownish liquid (>98% purity by HPLC).

### Example 2: Preparation of 4-(tert-butyl) benzoyl chloride (CIBB)

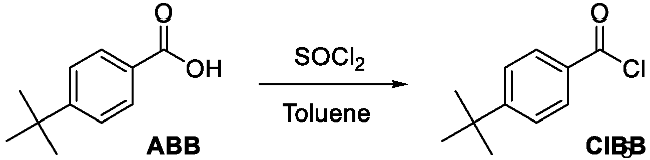

ABB (4-(tert-butyl)benzoic acid, 600 g, 3.366 mol) was suspended in 1800 mL of toluene in a 5 L reactor equipped with mechanical stirring, under inert atmosphere of N₂. The suspension was heated to 65-70 ºC. Thionyl chloride (SOCl₂, 800 g, 6.724 mol, 2 eq.) was added during 30-45 min into the reactor, keeping the temperature of the resulting mixture at 65-70 ºC. The reaction mixture was heated to 80-90 ºC during 20 min and then stirred at this temperature for 3 h, until no more gas release (SO₂ and HCl) was observed. Toluene and the remains of SOCl₂ were distilled under atmospheric pressure, obtaining a transparent bright yellow solution. Then, vacuum was applied progressively (T_{B} = 60 ºC) to further remove the two volatiles. The product was purified by distillation with a fractionating column (main fraction at T_{B} = 131-150 ºC, T_{V} = 110-112 ºC and 533-666 Pa (4-5 mmHg)), obtaining 551.3 g (83% yield) of CIBB as a colorless liquid.

### Example 3: preparation of 2-bromo-1-(4-methoxyphenyl)ethyl 4-(tert-butyl)benzoate (BBBF):

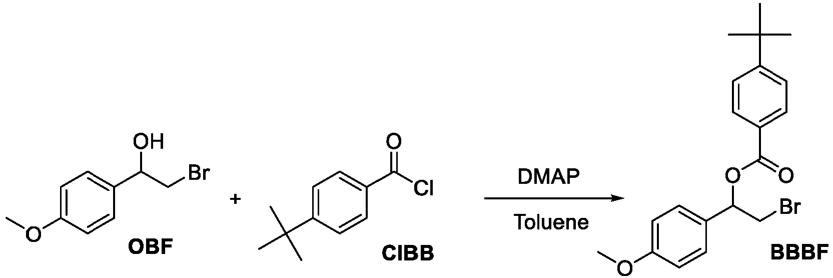

OBF (90.0 g, 0.389 mol) was dissolved in 840 mL of dry DCM in a 2 L reactor equipped with mechanical stirring and a dropping funnel with pressure compensation, under inert atmosphere of N₂. The mixture was cooled down to 10-15 ºC and DMAP 99% (4-dimethylaminopyridine, 57.25 g, 0.468 mol, 1.2 eq) was added keeping the temperature at 10-15 ºC. The mixture was stirred 15 min at 20-25 ºC observing complete dissolution. CIBB 98% (78.10 g, 0.389 mol) was added during 1 h into the reactor, keeping the temperature of the resulting mixture at 20-30 ºC. Once the addition was finished, the mixture was stirred for 2-3 h at 30-32 ºC, after which complete conversion of the starting material was observed by TLC. The mixture was cooled down to 10-15 ºC and the organic phase washed during 15 min with 2 x 280 mL of H₂O. The organic phase was filtered and concentrated under reduced pressure at 20-25 ºC for approximately 6 h, until constant weight, obtaining 156 g (quant. yield) of BBBF as a yellowish liquid (>95% purity by HPLC).

### Example 4: Preparation of 1-(4-methoxyphenylvinyl 4-(tert-butyl)benzoate (PREAVO-2)

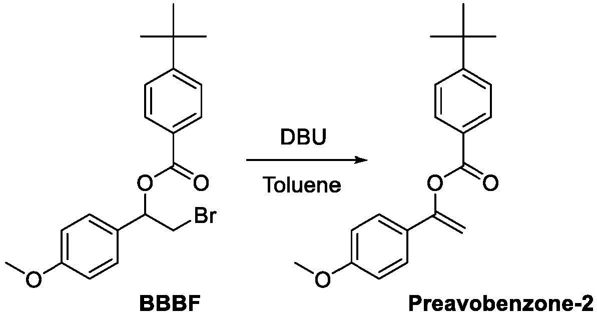

BBBF (178 g, 0.454 mol) was dissolved in 534 mL of toluene in a 5 L reactor equipped with mechanical stirring and prevented from light, under inert atmosphere of N₂. The solution was heated to 70-75 ºC. A solution of DBU (207.4 g, 1.362 mol, 3.0 eq.) in 478.4 mL of toluene was added during 2.5 h into the reactor, keeping the temperature of the resulting mixture at 70-75 ºC. Once the addition was finished, the reaction mixture was stirred for 5 h at 70-75 ºC, after which complete conversion of the starting material was observed by TLC. The mixture was cooled down to 20-25 ºC and 2620 mL of H₂O were added during 15 min, keeping the temperature at 20-25 ºC. The mixture was further stirred at that temperature for 15 min and let decant for 15 min. The organic phase was washed with 2 x 2620 mL of 0.5 M HCl and 2 x 2620 mL of H₂O. Each of these four consecutive extractions were performed adding the aqueous phase during 10-15 min at 20-25 ºC and mixing the two phases for 15 min at 20-25 ºC.

### Example 5: Crystallization of Preavobenzone-2: 1-(4-methoxyphenyl)vinyl 4-(tert-butyl)benzoate (PREAVO-2)

In the same reactor containing 140 g of crude Preavobenzone-2, 560 mL of EtOH were added. The mixture was heated to 50-55 ºC observing complete dissolution. The solution was cooled down to 45 ºC and the crystallization began. The suspension was further cooled down to 5-10 ºC during 1 h and then kept at 5-10 ºC fort 3 h. The crystallized product was filtered and washed with 56 mL of cold (0 ºC) EtOH. The solid was dried under vacuum in the oven at 40 ºC during approximately 15 h, until constant weight, obtaining 100.6 g (75% yield) of Preavobenzone-2 as a white crystalline solid (>99% purity by HPLC).

### Citation List

### Patent Literature

WO2006/100225A1

### Non-Patent Literature

A. Gallardo et al., A new strategy for photoprotection?", Photochemical & Photobiological Sciences (2010), 9(4), 530-534
Noriko Okamoto et al, , Regio- and Stereoselective Multisubstituted Enol Ester Synthesis" JOC, 2011, vol. 76, pp. 9133-9138
Qi Xinxin et al.; "Palladium/aluminium-cocatalyzed carbonlylative synthesis of 2-chloroethyl benzoates from epoxides and aryl iodides", Journal of Organometallic Chemistry, 2020, vol. 910, 121114

## Claims

1. A process for preparing a compound of formula (I), a pharmaceutically or a cosmetically acceptable salt thereof, or a stereoisomer of any of them or mixtures thereof, 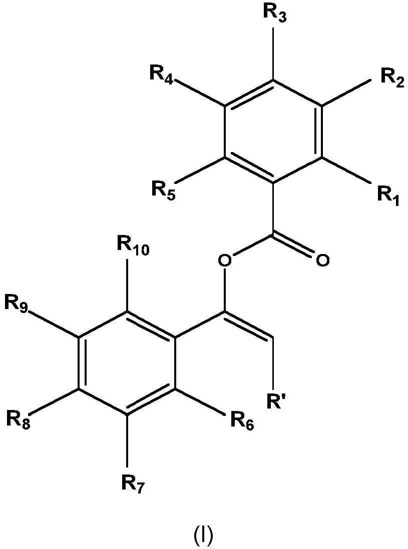 wherein:
R' is selected from the group consisting of H; (C₁-C₆)-alkyl, and (C₃-C₆)-cycloalkyl;
R₁, R₂, R₄, R₅, R₆, R₇, R₉, and R₁₀ are a radical independently selected from the group consisting of H, hydroxy, amino, (C₁-C₆)-alkyl, (C₁-C₆)-alkoxy, C₁-C₆)-alkylamino, and (C₁-C₆)-dialkylamino; and
R₃ and R₈ are independently selected form the group consisting of (C₁-C₆)-alkyl, (C₁-C₆)-alkoxy, hydroxy, amino, (C₁-C₆)-alkylamino, and (C₁-C₆)-dialkylamino;
the process comprising a step of reacting a compound of formula (II) or an appropriate salt thereof; 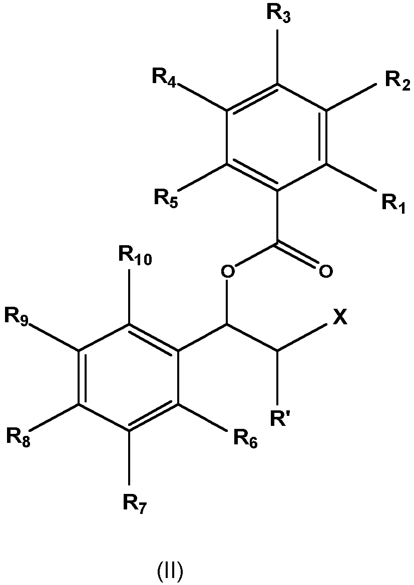
wherein: R', R₁₋R₁₀ are as defined in compound of formula (I); and X is a halogen selected from the group consisting of Cl, Br, and I;
with a non-nucleophilic strong base, in the presence of an appropriate solvent.

2. The process according to claim 1, wherein the no-nucleophilic base is a selected from 1,8-diazabicyclo[5.4.0]undec-7-ene (Illa) and 1,1,3,3-tetramethylguanidine (IIIb).

3. The process according to any of the claims 1-2, wherein in compound of formula (II) X is bromine.

4. The process according to any of the claims 1-3, wherein in compound of formula (I) and in compound of formula (II) R', R₁, R₂, R₄, R₅, R₆, R₇, R₉, and R₁₀ are H; R₃ is tert-butyl and R₈ is methoxyl.

5. The process according to any of the claims 1-4, wherein the solvent is selected from the group consisting of a (C₆-C₈)-aromatic hydrocarbons and (C₁-C₃)-chlorine containing solvents.

6. The process according to any of the claims 1-5, wherein the process comprises previously reacting a compound of formula (IV) with either a compound of formula (V) or a stereoisomer thereof or mixtures thereof, in the presence of an appropriate solvent to yield compound of formula (II). 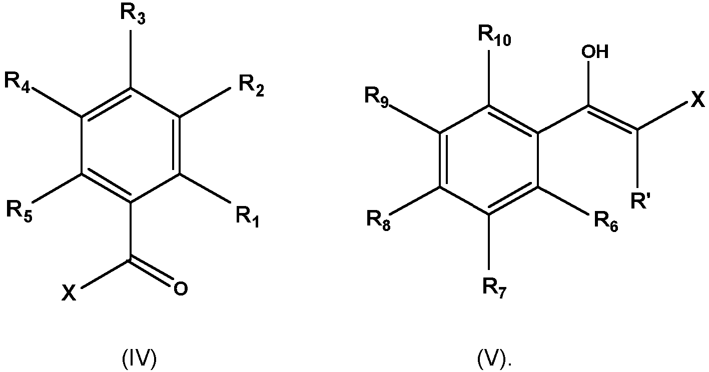 wherein: R', R₁₋R₁₀, are as defined in compound of formula (I) and X is a halogen selected from the group consisting of Cl, Br, and I.

7. The process according to claim 6, wherein the process is carried out in the presence of a coupling agent capable of intervening in the conversion of an acyl halide to an ester.

8. The preparation process according to any of the claims 6-7, wherein the process further comprises a previous step comprising reacting a compound of formula (VI) with a halogenating agent in the presence of an appropriate solvent to yield the compound of formula (IV). 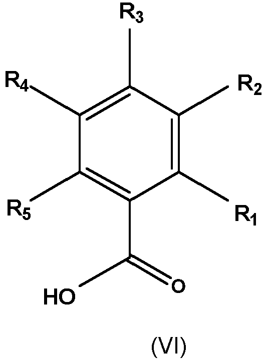
R₁, R₂, R₄, and R₅, are a radical independently selected from the group consisting of H, hydroxy, amino, (C₁-C₆)-alkyl, (C₁-C₆)-alkoxy, C₁-C₆)-alkylamino, and (C₁-C₆)-dialkylamino; and
R₃ is selected form the group consisting of (C₁-C₆)-alkyl, (C₁-C₆)-alkoxy, hydroxy, amino, (C₁-C₆)-alkylamino, and (C₁-C₆)-dialkylamino.

9. The process according to claim 7, wherein the halogenating agent is selected from the group consisting of: SO₂Cl₂, SOCl₂, PBr₃, and PBr₅.

10. The process according to any of the claims 6-7, wherein the process further comprises a previous step comprising reacting a compound of formula (VII) with a reducing agent in the presence of a solvent to yield the compound of formula (V), 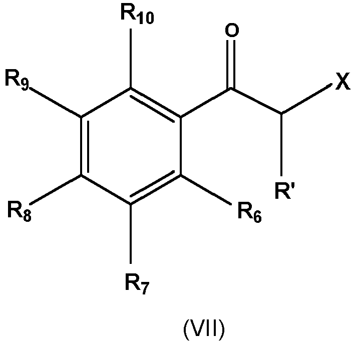 wherein:
R' is H;
R₆, R₇, R₉, and R₁₀ are a radical independently selected from the group consisting of H, hydroxy, amino, (C₁-C₆)-alkyl, (C₁-C₆)-alkoxy, C₁-C₆)-alkylamino, and (C₁-C₆)-dialkylamino;
R₈ is independently selected form the group consisting of (C₁-C₆)-alkyl, (C₁-C₆)-alkoxy, hydroxy, amino, (C₁-C₆)-alkylamino, and (C₁-C₆)-dialkylamino; and
X is a halogen selected from the group consisting of Cl, Br, and I.

11. The process according to claim 10, wherein the reducing agent is selected from sodium borohydride and lithium aluminium hydride.

12. A compound of formula (II) or an appropriate salt thereof: 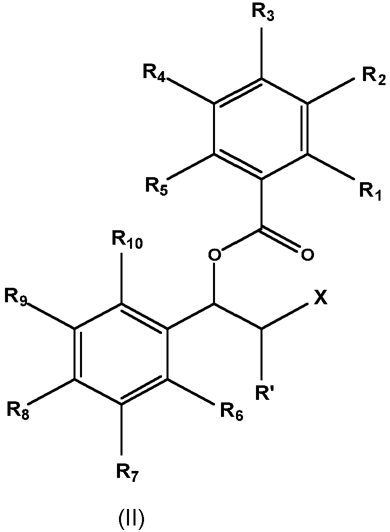 wherein:
R' is selected from the group consisting of **H,** (C₁-C₆)-alkyl, and (C₃-C₆)-cycloalkyl;
R₁, R₂, R₄, R₅, R₆, R₇, R₉, and R₁₀ are a radical independently selected from the group consisting of **H,** hydroxy, amino, (C₁-C₆)-alkyl, (C₁-C₆)-alkoxy, (C₁-C₆)-alkylamino, and (C₁-C₆)-dialkylamino;
R₃ and R₈ are independently selected form the group consisting of (C₁-C₆)-alkyl, (C₁-C₆)-alkoxy, hydroxy, amino, (C₁-C₆)-alkylamino, and (C₁-C₆)-dialkylamino; and
X is a halogen selected from the group consisting of Cl, Br, and i.

13. The compound of formula (II) according to claim 12, wherein X is bromine.

14. The compound of formula (II) according to any of the claims 12-13, wherein R₃ is tert-butyl and R₈ is methoxyl.

15. The compound of formula (II) according to any of the claims 12-14, wherein R', R₁, R₂, R₄, R₅, R₆, R₆, R₇, R₉, and R₁₀ are H.

## Patentansprüche

1. Ein Verfahren zur Herstellung von einer Verbindung der Formel (I), einem pharmazeutisch oder kosmetisch akzeptablen Salz davon oder einem Stereoisomer eines davon oder Mischungen davon, 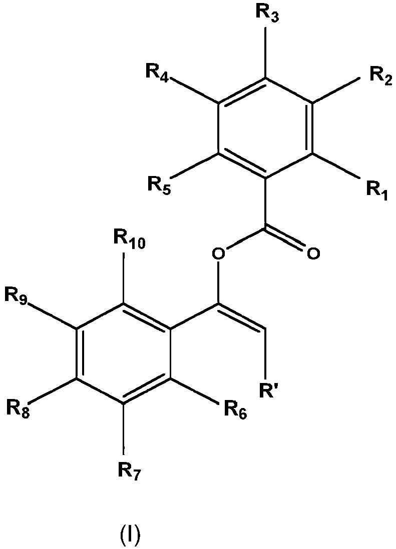 wobei:
R' ausgewählt ist aus der Gruppe bestehend aus H; (C₁-C₆)-Alkyl und (C₃-C₆)-Cycloalkyl;
R₁, R₂, R₄, R₅, R₆, R₇, R₉ und R₁₀ ein Rest sind, der unabhängig voneinander ausgewählt ist aus der Gruppe bestehend aus H, Hydroxy, Amino, (C₁-C₆)-Alkyl, (C₁-C₆)-Alkoxy, C₁-C₆)-Alkylamino und (C₁-C₆)-Dialkylamino; und
R₃ und R₈ unabhängig voneinander ausgewählt sind aus der Gruppe bestehend aus (C₁-C₆)-Alkyl, (C₁-C₆)-Alkoxy, Hydroxy, Amino, (C₁-C₆)-Alkylamino und (C₁-C₆)-Dialkylamino;
wobei das Verfahren einen Schritt des Umsetzens einer Verbindung der Formel (II) oder eines geeigneten Salzes davon umfasst; 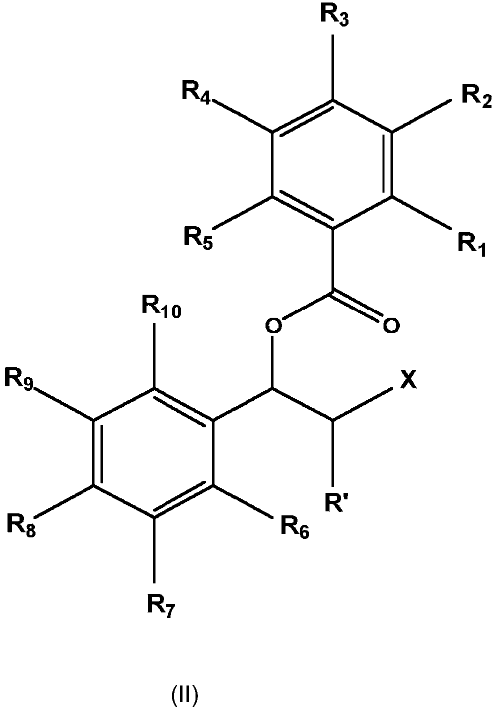
wobei: R', R₁-R₁₀ wie bei der Verbindung der Formel (I) definiert sind; und X ein Halogen ist, das aus der Gruppe ausgewählt ist, die aus Cl, Br und I besteht;
mit einer nicht-nukleophilen starken Base in Gegenwart eines geeigneten Lösungsmittels.

2. Das Verfahren nach Anspruch 1, wobei die nicht-nukleophile Base ausgewählt ist aus 1,8-Diazabicyclo[5.4.0]undec-7-en (Illa) und 1,1,3,3-Tetramethylguanidin (lllb).

3. Das Verfahren nach einem der Ansprüche 1 bis 2, wobei in der Verbindung der Formel (II) X = Brom ist.

4. Das Verfahren nach einem der Ansprüche 1 bis 3, wobei in der Verbindung der Formel (I) und in der Verbindung der Formel (II) R', R ₁, R₂, R₄, R₅, R₆, R₇, R₉ und R₁₀ = H sind; R₃ = tert-Butyl ist und R₈ = Methoxyl ist.

5. Das Verfahren nach einem der Ansprüche 1 bis 4, wobei das Lösungsmittel aus der Gruppe ausgewählt ist, die aus einem aromatischen (C₆-C₈)-Kohlenwasserstoff und (C₁-C₃)-chlorhaltigen Lösungsmitteln besteht.

6. Das Verfahren nach einem der Ansprüche 1 bis 5, wobei das Verfahren das vorherige Umsetzen einer Verbindung der Formel (IV) mit entweder einer Verbindung der Formel (V) oder einem Stereoisomer davon oder Mischungen davon in Gegenwart eines geeigneten Lösungsmittels umfasst, um die Verbindung der Formel (II) zu ergeben. 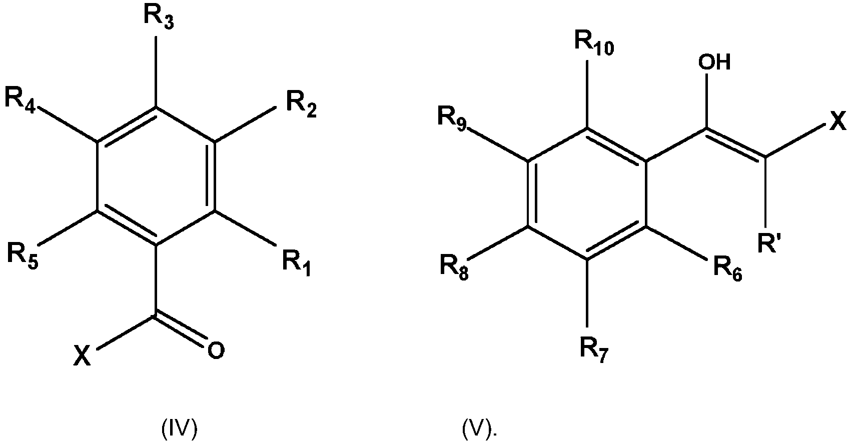 wobei: R', R₁-R₁₀, wie bei der Verbindung der Formel (I) definiert sind und X ein Halogen ist, das aus der Gruppe ausgewählt ist, die aus Cl, Br und I besteht.

7. Das Verfahren nach Anspruch 6, wobei das Verfahren in Gegenwart eines Kupplungsmittels durchgeführt wird, das in der Lage ist, in die Umwandlung eines Säurehalogenids in einen Ester einzugreifen.

8. Das Herstellungsverfahren nach einem der Ansprüche 6 bis 7, wobei das Verfahren ferner einen vorhergehenden Schritt umfasst, der das Umsetzen einer Verbindung der Formel (VI) mit einem Halogenierungsmittel in Gegenwart eines geeigneten Lösungsmittels umfasst, um die Verbindung der Formel (IV) zu ergeben. 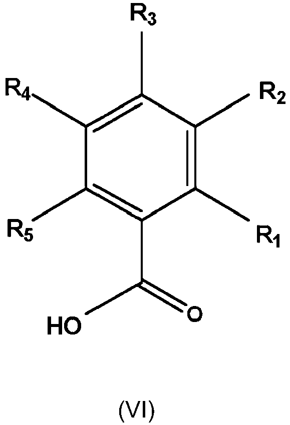
R₁, R₂, R₄ und R₅ sind ein Rest, der unabhängig voneinander ausgewählt ist aus der Gruppe bestehend aus H, Hydroxy, Amino, (C₁-C₆)-Alkyl, (C₁-C₆)-Alkoxy, (C₁-C₆)-Alkylamino und (C₁-C₆)-Dialkylamino; und
R₃ ist ausgewählt aus der Gruppe bestehend aus (C₁-C₆)-Alkyl, (C₁-C₆)-Alkoxy, Hydroxy, Amino, (C₁-C₆)-Alkylamino und (C₁-C₆)-Dialkylamino.

9. Das Verfahren nach Anspruch 7, wobei das Halogenierungsmittel ausgewählt ist aus der Gruppe bestehend aus: SO₂Cl₂, SOCl₂, PBr₃ und PBr_{5.}

10. Das Verfahren nach einem der Ansprüche 6 bis 7, wobei das Verfahren ferner einen vorhergehenden Schritt umfasst, der das Umsetzen einer Verbindung der Formel (VII) mit einem Reduktionsmittel in Gegenwart eines Lösungsmittels umfasst, um die Verbindung der Formel (V) zu ergeben, 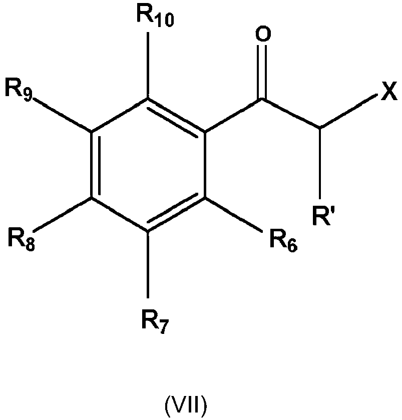 wobei:
R' = H ist;
R₆, R₇, R₉ und R₁₀ ein Rest sind, der unabhängig voneinander ausgewählt ist aus der Gruppe bestehend aus H, Hydroxy, Amino, (C₁-C₆)-Alkyl, (C₁-C₆)-Alkoxy, (C₁-C₆)-Alkylamino und (C₁-C₆)-Dialkylamino;
R₈ unabhängig ausgewählt ist aus der Gruppe bestehend aus (C₁-C₆)-Alkyl, (C₁-C₆)-Alkoxy, Hydroxy, Amino, (C₁-C₆)-Alkylamino und (C₁-C₆)-Dialkylamino; und
X ein Halogen ausgewählt aus der Gruppe bestehend aus Cl, Br und I ist.

11. Das Verfahren nach Anspruch 10, wobei das Reduktionsmittel ausgewählt aus Natriumborhydrid und Lithiumaluminiumhydrid ist.

12. Eine Verbindung der Formel (II) oder ein geeignetes Salz davon: 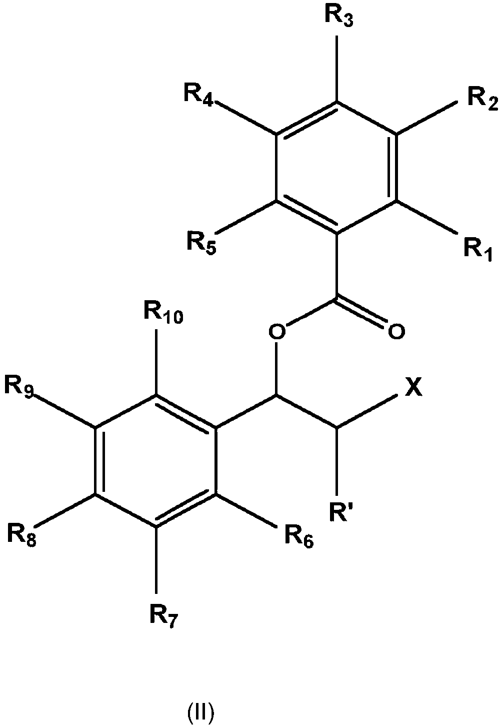 wobei:
R' ausgewählt ist aus der Gruppe bestehend aus H, (C₁-C₆)-Alkyl und (C₃-C₆)-Cycloalkyl;
R₁, R₂, R₄, R₅, R₆, R₇, R₉ und R₁₀ ein Rest sind, der unabhängig voneinander ausgewählt ist aus der Gruppe bestehend aus H, Hydroxy, Amino, (C₁-C₆)-Alkyl, (C₁-C₆)-Alkoxy, (C₁-C₆)-Alkylamino und (C₁-C₆)-Dialkylamino;
R₃ und R₈ unabhängig voneinander ausgewählt sind aus der Gruppe bestehend aus (C₁-C₆)-Alkyl, (C₁-C₆)-Alkoxy, Hydroxy, Amino, (C₁-C₆)-Alkylamino und (C₁-C₆)-Dialkylamino; und
X ein Halogen ausgewählt aus der Gruppe bestehend aus Cl, Br und I ist.

13. Die Verbindung der Formel (II) nach Anspruch 12, wobei X = Brom ist.

14. Die Verbindung der Formel (II) nach einem der Ansprüche 12 bis 13, wobei R₃ = tert-Butyl ist und R₈ = Methoxyl ist.

15. Die Verbindung der Formel (II) nach einem der Ansprüche 12 bis 14, wobei R', R₁, R₂, R₄, R₅, R₆, R₆, R₇, R₉ und R₁₀ = H sind.

## Revendications

1. Un procédé de préparation d'un composé de formule (I), d'un sel pharmaceutiquement ou cosmétiquement acceptable de celui-ci, ou d'un stéréoisomère de l'un quelconque de ceux-ci ou de mélanges de ceux-ci, 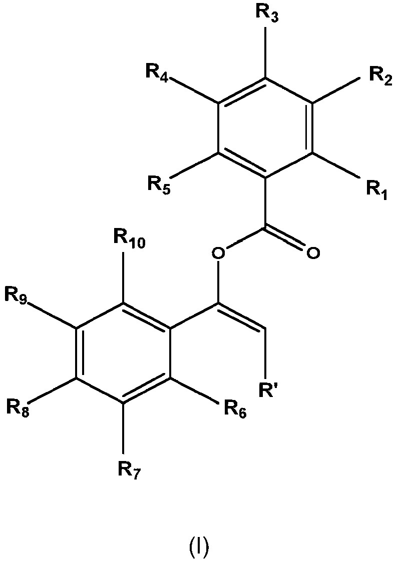 dans laquelle :
R' est choisi dans le groupe constitué par H ; alkyle en (C₁-C₆) et cycloalkyle en (C₃-C₆) ;
R₁, R₂, R₄, R₅, R₆, R₇, R₉ et R₁₀ sont un radical choisi indépendamment dans le groupe constitué par H, hydroxy, amino, alkyle en (C₁-C₆), alcoxy en (C₁-C₆), alkylamino en (C₁-C₆) et dialkylamino en (C₁-C₆) ; et
R₃ et R₈ sont choisis indépendamment dans le groupe constitué par les groupes alkyle en (C₁-C₆), alcoxy en (C₁-C₆), hydroxy, amino, alkylamino en (C₁-C₆) et dialkylamino en (C₁-C₆) ;
le procédé comprenant une étape consistant à faire réagir un composé de formule (II) ou un sel approprié de celui-ci ; 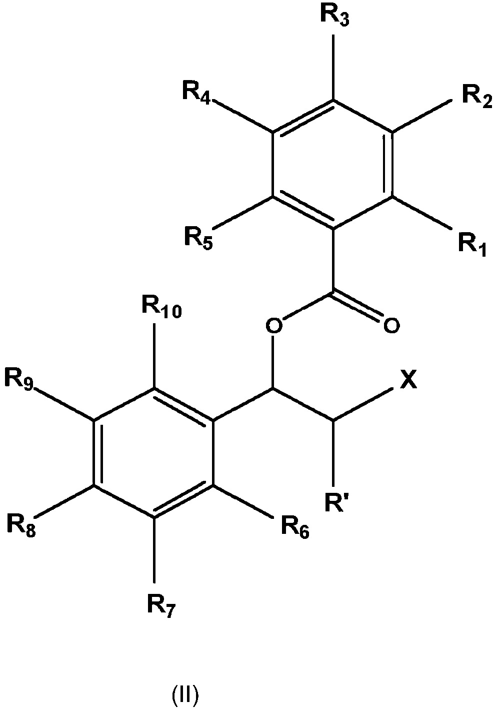
dans laquelle : R', R₁-R₁₀ sont tels que définis dans le composé de formule (I) ; et X est un halogène choisi dans le groupe constitué par Cl, Br et I ;
avec une base forte non nucléophile, en présence d'un solvant approprié.

2. Le procédé selon la revendication 1, dans lequel la base non nucléophile est une choisie parmi le 1,8-diazabicyclo[5.4.0]undéc-7-ène (Illa) et la 1,1,3,3-tétraméthylguanidine (lllb).

3. Le procédé selon l'une quelconque des revendications 1 à 2 dans lequel, dans le composé de formule (II) X est le brome.

4. Le procédé selon l'une quelconque des revendications 1 à 3, dans lequel dans le composé de formule (I) et dans le composé de formule (II), R', R ₁, R ₂, R ₄, R ₅, R ₆, R ₇, R ₉ et R₁₀ sont H ; R₃ est le tert-butyle et R₈ est le méthoxyle.

5. Le procédé selon l'une quelconque des revendications 1 à 4, dans lequel le solvant est choisi dans le groupe constitué des hydrocarbures aromatiques en (C₆-C₈) et des solvants contenant du chlore en (C₁-C₃).

6. Le procédé selon l'une quelconque des revendications 1 à 5, dans lequel le procédé comprend la réaction préalable d'un composé de formule (IV) avec soit un composé de formule (V), soit un stéréoisomère de celui-ci, soit des mélanges de ceux-ci, en présence d'un solvant approprié pour donner le composé de formule (II). 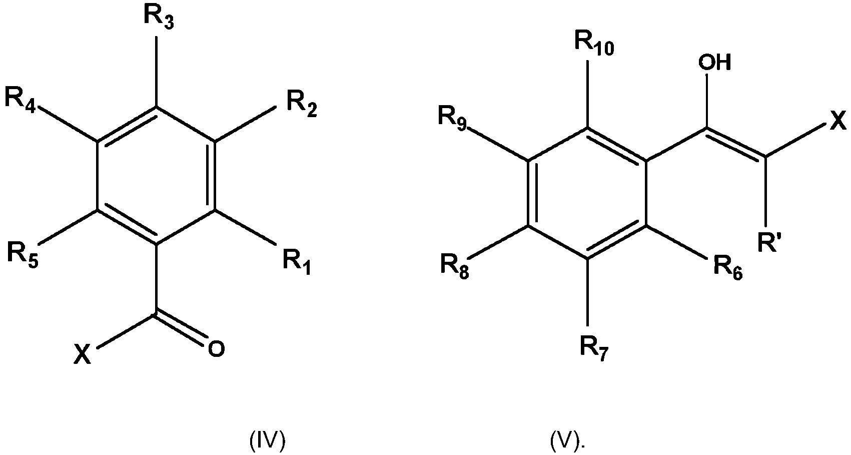 dans lesquelles : R', R₁-R₁₀, sont tels que définis dans le composé de formule (I) et X est un halogène choisi dans le groupe constitué par Cl, Br et I.

7. Le procédé selon la revendication 6, dans lequel le procédé est mis en œuvre en présence d'un agent de couplage capable d'intervenir dans la conversion d'un halogénure d'acyle en un ester.

8. Le procédé de préparation selon l'une quelconque des revendications 6 à 7, dans lequel le procédé comprend en outre une étape précédente comprenant la réaction d'un composé de formule (VI) avec un agent d'halogénation en présence d'un solvant approprié pour donner le composé de formule (IV). 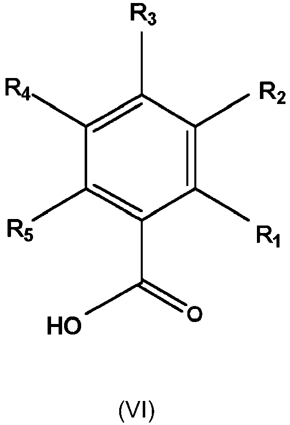
R₁, R₂, R₄ et R₅ sont un radical choisi indépendamment dans le groupe constitué par H, hydroxy, amino, alkyle en (C₁-C₆), alcoxy en (C₁-C₆), alkylamino en (C₁-C₆) et dialkylamino en (C₁-C₆) ; et
R₃ est choisi dans le groupe constitué par alkyle en (C₁-C₆), alcoxyle en (C₁-C₆), hydroxy, amino, alkylamino en (C₁-C₆) et dialkylamino en (C₁-C₆).

9. Le procédé selon la revendication 7, dans lequel l'agent d'halogénation est choisi dans le groupe constitué par : SO₂Cl₂, SOCl₂, PBr₃, et PBr₅.

10. Le procédé selon l'une quelconque des revendications 6 à 7, dans lequel le procédé comprend en outre une étape précédente comprenant la réaction d'un composé de formule (VII) avec un agent réducteur en présence d'un solvant pour donner le composé de formule (V), 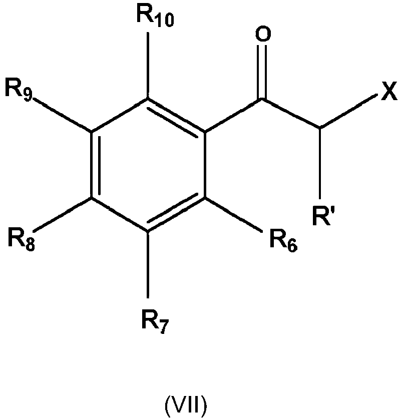 dans laquelle :
R' est H;
R₆, R₇, R₉ et R₁₀ sont un radical choisi indépendamment dans le groupe constitué par H, hydroxy, amino, alkyle en (C₁-C₆), alcoxy en (C₁-C₆), alkylamino en (C₁-C₆) et dialkylamino en (C₁-C₆) ;
R₈ est choisi indépendamment dans le groupe constitué par alkyle en (C₁-C₆), alcoxy en (C₁-C₆), hydroxy, amino, alkylamino en (C₁-C₆) et dialkylamino en (C₁-C₆) ; et
X est un halogène choisi dans le groupe constitué par Cl, Br et I.

11. Le procédé selon la revendication 10, dans lequel l'agent réducteur est choisi parmi le borohydrure de sodium et l'hydrure de lithium et d'aluminium.

12. Un composé de formule (II) ou un sel approprié de celui-ci : 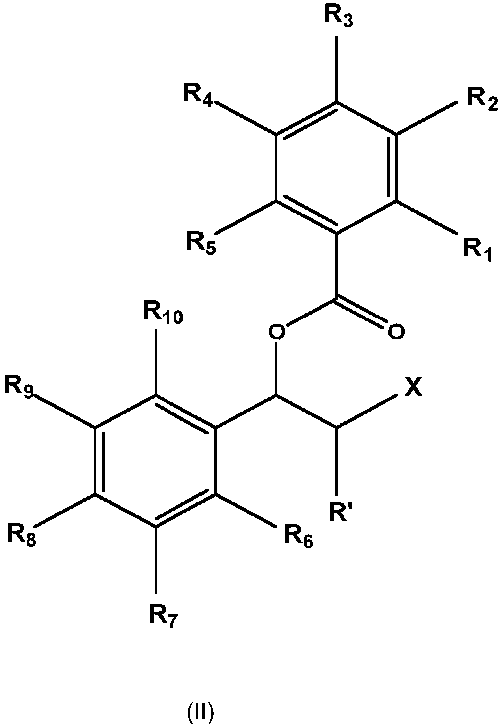 dans laquelle :
R' est choisi dans le groupe constitué par H, alkyle en (C₁-C₆) et cycloalkyle en (C₃-C₆) ;
R₁, R₂, R₄, R₅, R₆, R₇, R₉ et R₁₀ sont un radical choisi indépendamment dans le groupe constitué par H, hydroxy, amino, alkyle en (C₁-C₆), alcoxy en (C₁-C₆), alkylamino en (C₁-C₆) et dialkylamino en (C₁-C₆) ;
R₃ et R₈ sont choisis indépendamment dans le groupe constitué par les groupes alkyle en (C₁-C₆), alcoxy en (C₁-C₆), hydroxy, amino, alkylamino en (C₁-C₆) et dialkylamino en (C₁-C₆) ; et
X est un halogène choisi dans le groupe constitué par Cl, Br et I.

13. Le composé de formule (II) selon la revendication 12, dans laquelle X est le brome.

14. Le composé de formule (II) selon l'une quelconque des revendications 12 à 13, dans laquelle R₃ est le tert-butyle et R₈ est le méthoxyle.

15. Le composé de formule (II) selon l'une quelconque des revendications 12 à 14, dans laquelle R', R ₁, R ₂, R ₄, R ₅, R_{6,} R₆, R₇, R₉ et R₁₀ sont H.
